# EUROPEAN PATENT SPECIFICATION

(11) **EP 2 872 046 B1**
(45) Date of publication and mention of the grant of the patent: **08.05.2019**
(21) Application number: 13740371.3
(22) Date of filing: 10.07.2013
(51) Int. Cl.: A61B 10/02, A61B 17/3207, A61B 18/14, A61B 5/00, A61B 10/04

(54) **MEDICAL DEVICE FOR USE IN TISSUE CHARACTERIZATION AND TREATMENT**
MEDIZINISCHE VORRICHTUNG ZUR VERWENDUNG IN EINER GEWEBEKENNZEICHNUNG UND -BEHANDLUNG
DISPOSITIF MÉDICAL DESTINÉ À LA CARACTÉRISATION ET AU TRAITEMENT DES TISSUS

(30) Priority: 12.07.2012 US 201213547950; 14.11.2012 US 201213676993; 03.04.2013 US 201313856340
(43) Date of publication of application: 20.05.2015
(73) Proprietor: Dune Medical Devices Ltd., 3088900 Caesarea (IL)
(72) Inventor: HASHIMSHONY, Dan, 37017 Pardes Hana (IL); COHEN, Gil, 9782224 Jerusalem (IL); GELTNER, Iddo, 40500 Even Yehuda (IL); AHARONOWITZ, Gal, 4491000 Moshav Gan Haim (IL)
(74) Representative: Becker Kurig Straus
(86) International application number: PCT/IB2013/055661
(87) International publication number: WO 2014/009893

(56) References cited:
- WO-A1-98/12968
- WO-A1-99/44506
- WO-A1-2012/092016
- WO-A1-2012/092016
- WO-A2-01/82998
- WO-A2-2009/010960
- WO-A2-2009/010960
- WO-A2-2009/010960
- US-A1- 2003 050 574
- US-A1- 2004 255 739
- US-A1- 2005 203 419
- US-A1- 2008 214 955
- US-A1- 2008 214 955

## Description

### FIELD OF THE INVENTION

This invention relates to medical devices for use in tissue characterization and treatment.

### BACKGROUND

Techniques for identifying abnormal (e.g. tumorous) cells in a biological tissue are generally known. Such techniques include those utilizing determination of the electrical properties of a tissue, for example, by determination of electrical impedance or dielectric constants. Some kinds of tumors can be identified by determining differences in the measured electrical properties of the tissue. The identified and located region of abnormal tissue can then be treated and/or removed from the body

Various types of tissue characterization sensor and its integration with a tissue treatment/removal tool are described in the following patent publications, all assigned to the assignee of the present application: US2003138378, WO2006103665, WO2007015255, WO2009010960, US6813515 and US7184824.

Also, various techniques are known for removing a certain tissue specimen from a tissue mass. These techniques are disclosed for example in US 6,689,145 and US 7,122,011.

WO 2009/010960 describes a hollow probe for tissue characterisation comprising an elongated carrier with tissue characterisation sensors, thereby enabling consequent treatment of abnormal tissue specimen by a treatment tool

### GENERAL DESCRIPTION

There is a need in the art to facilitate precise location and determination of a volume of a tissue specimen (e.g. abnormal tissues) to be treated (e.g. removed), and adjustment of a treatment volume to the determined volume of the abnormal tissue specimen.

The present invention solves the above problems by providing a novel medical device for use in tissue characterization and treatment with a device according to claim 1. The device comprises a tissue characterization probe comprising an elongated carrier carrying an array (one- or two-dimensional array) of tissue characterization sensors which are located at least within a distal portion of the carrier and are arranged in a spaced-apart relationship with known distances between them along at least a longitudinal axis of the carrier (and possibly also along at least a part of circumference of the carrier). Each tissue characterization sensor is configured and operable for generating a tissue characterization signal corresponding to a tissue mass at a location of the sensor. Hence, during progression of the probe through a tissue mass each of the sensors generates tissue characterization signals from successive locations thereof within the tissue mass enabling to locate an abnormal tissue inside said tissue mass, based on the characterization signals from the sensors in the array. The elongated carrier has two integral portions including the distal portion and a hollow portion extending between a proximal end of the carrier and the distal portion. The carrier is further configured for passing a treatment tool through the hollow portion thereof and enabling at least a part of the treatment tool to project from the hollow portion and extend along the distal portion. This configuration of the device enables consequent treatment of the abnormal tissue by a treatment tool carried by the carrier.

The treatment tool suitable to be used with the device of the invention may be configured for carrying out at least one of the following: biopsy, cutting, delivering physical treatment, delivering treatment medication, diagnostics. In some embodiments of the invention, the treatment tool is carried by (e.g. mounted on or inside) the characterization probe carrier. In some embodiments, the treatment tool may be selectively shiftable between its inoperative position being located substantially entirely inside the carrier and its operative position projecting by at least one tissue treating portion towards outside the carrier. The dimension of the tissue treating portion(s) projectable from the carrier, and possibly also location of the tissue treating portion(s) with respect to the carrier can be controllably varied.

According to the invention, the distal portion is configured as a trough like member thereby enabling concurrent alignment of a distal portion of the treatment tool at one side of the distal portion of the carrier and the array of tissue characterization sensors at opposite side of the distal portion of the carrier, to the same segment of the tissue mass.

The device may comprise a handle portion connectable to or integral with the proximal end of the carrier. The handle portion may be configured for engaging with a handle of the treatment tool when the treatment tool is inserted into the hollow portion of the carrier.

According to some embodiments of the invention, the device comprises a movement mechanism located at the proximal end of the carrier, and being configured and operable to enable relative displacement between the carrier and the treatment tool. The movement mechanism may be located inside the handle portion of the carrier.

The movement mechanism may comprise a registration assembly to define a reference position for the array of tissue characterization sensors with respect to a certain reference plane defined by the device, thereby enabling to monitor repositioning of the tissue characterization sensors caused by movement of the carrier with respect to the reference plane. The registration assembly may comprise a sensing unit configured and operable to identify a position of the sensors array relatively to said reference plane and to generate data indicative thereof.

The movement mechanism may be located at the proximal end of the carrier, and the reference plane is defined by the handle.

The registration assembly may comprise an L-like shaped bracket which by its one arm of a given length is movably connected to the handle and by its other arm is connected to the carrier. The reference plane may be defined by a distal edge of the handle, thereby enabling to monitor repositioning of the tissue characterization sensors caused by movement of the bracket with respect to the reference plane.

There are several options for configuring the movement mechanism. For example, the movement mechanism may comprise a locking system configured to maintain the sensor array at fixed and locked positions. Further, the movement mechanism may comprise a slider configured to selectively displace the treatment tool between inoperative and operative positions. The movement mechanism may be configured to convert a rotational movement into the linear relative displacement between the carrier and the treatment tool. The movement mechanism may comprise rack and pinion configuration.

According to some embodiments of the invention, the device may be configured as a two-part assembly comprising a first assembly and a second assembly, the first and second assemblies being removably attachable to one another.

The first assembly may carry the tissue characterization probe and the second assembly carries the treatment tool. Alternatively, both of the tissue characterization probe and the treatment tool may be carried by either the first assembly or the second assembly.

According to another broad aspect of the invention, there is provided a medical device for use in tissue characterization and treatment, the device comprises a tissue characterization probe comprising an elongated carrier having a hollow portion for passing therein a treatment tool, an array of tissue characterization sensors which are located on the carrier at least within a distal portion thereof and are arranged in a spaced-apart relationship with known distances between them enabling to locate an abnormal tissue inside a tissue mass during progression of the probe through the tissue mass based on tissue characterization signals from the array of sensors and to perform consequent treatment of a tissue specimen by the treatment tool; and a movement mechanism located at the proximal end of the carrier, and being configured to enable relative displacement between the carrier and the treatment tool. As described above, the distal portion is configured as a trough like member enabling concurrent alignment of the distal portion of the treatment tool and the array of tissue characterization sensors with the same segment of the tissue mass. As also mentioned above, the treatment tool may be selectively shiftable between its inoperative position being located substantially entirely inside the hollow portion of the carrier and its operative position projecting by its at least one tissue treating portion towards the distal portion of the carrier.

According to some embodiments, the treatment tool and the carrier are configured to enable the treatment tool to be selectively shiftable between its inoperative position being located substantially entirely inside the hollow portion of the carrier and its operative position projecting by its at least one tissue treating portion towards outside the carrier when a part of the treatment tool extends along the distal portion of the carrier.

Preferably, the device, of either one of the above-described embodiments, comprises a control unit configured for receiving and analyzing tissue characterizing signals from each of all the sensors and utilizing data indicative of the respective sensors' location for determining the location, and optionally the dimension, of an abnormal tissue specimen, thereby enabling consequent treatment of the abnormal tissue specimen by the treatment tool. The control unit preferably comprises a graphical user interface configured for presenting information related to the signals received from all the sensors, thereby providing an operator with information regarding the tissue type at the locations of the sensors, and facilitating analysis of the location and extent of the tissue to be treated.

As indicated above, the tissue characterization probe of the invention includes an array (one- or two-dimensional array) of tissue characterization sensors. In the simplest example, this is a one-dimensional array of spaced-apart sensors along the longitudinal axis of the probe. Thus, for a given position of the probe with respect to tissue under measurement (the tissue into which the probe is inserted), the array of sensors arranged in a predetermined fashion actually presents an array of measurement locations/sites. According to the invention, such an array/matrix of the measurement locations (sensors' positions) is displayed together with corresponding measured data.

According to another aspect of the invention, there is provided a system for use in tissue characterization and treatment, the system comprising the above described medical device and a control unit connectable thereto. The control unit comprises a graphical user interface configured for presenting information related to the signals received from all the sensors. The presented information includes a corresponding array of measured data in association with the array of measurement locations. The array of measured data is indicative of the signals received from the array of the sensors respectively, thereby providing an operator with information regarding the tissue type at the locations of the sensors, i.e. a measured tissue profile along a tissue mass aligned with the array of sensors, and facilitating analysis of the location and extent of the tissue to be treated.

The measured data may be represented by one of the following:
(1) a respective value corresponding to a tissue property being sensed by the respective sensor, an array of the values defining the profile of the measured tissue property within a region aligned with the array of sensors;
(2) a respective figure having a geometry or size corresponding to a tissue property being sensed by the respective sensor, an array of the figures defining the profile of the measured tissue property within a region aligned with the array of sensors;
(3) respective figure and value, a geometry or size of the figure and the value corresponding to a tissue property being sensed by the respective sensor, an array of the figures and values defining the profile of the measured tissue property within a region aligned with the array of sensors; for example, the figures corresponding to the same measured data have the same color and figures corresponding to different measured data have different colors.

The graphical user interface may be configured for displaying data indicative of a threshold value for the tissue property being measured.

The array of measurement locations may be presented in the form of the array of measured data each being represented by a colored region, such that the regions corresponding to the same measured data have the same color and regions corresponding to different measured data have different colors.

The control unit may be configured and operable for receiving from one or more imaging modalities imaging data of the tissue characterization probe position with respect to tissue being measured, and merging the imaging data with the measured data, to thereby identify locations of the sensors on a merged image.

According to yet another aspect of the invention, there is provided a control unit for monitoring measurements of tissue properties, the control unit comprising a graphical user interface configured for presenting information related to signals received from an array of sensors arranged in a predetermined spaced-apart relationship along a measurement axis, the presented information being in the form of an array of locations spaced-apart along an axis corresponding to the array of said sensors, and a corresponding array of measured data in association with the array of said locations, said array of measured data being indicative of signals received from the array of the sensors respectively, thereby providing an operator with information regarding a profile of a measured tissue profile along a tissue mass aligned with the array of sensors.

According to yet further aspect of the invention, there is provided a graphical user interface configured for presenting information related to signals received from an array of sensors arranged in a predetermined spaced-apart relationship along a measurement axis, the presented information being in the form of an array of locations spaced-apart along an axis corresponding to the array of said sensors, and a corresponding array of measured data in association with the array of said locations, said array of measured data being indicative of signals received from the array of the sensors respectively, thereby providing an operator with information regarding a profile of measured tissue properties along a tissue mass aligned with the array of sensors.

### BRIEF DESCRIPTION OF THE DRAWINGS

In order to understand the invention and to see how it may be carried out in practice, embodiments will now be described, by way of non-limiting example only, with reference to the accompanying drawings, in which:
**Figs. 1A and 1B** are schematic illustrations of examples of a medical device of the present invention;
**Fig. 1C** shows a cross sectional view of a probe of Fig. 1B.
**Figs. 2 to 6** show five examples, respectively, of the device configuration for both the tissue characterization and removal of a tissue specimen;
**Figs. 7A1-7A2** and **7B1-7B2** show two examples, respectively, of the device configuration utilizing probes configured generally as shown in Fig. 1A and 1B, where a treatment tool can be applied to the desired location, identified by the tissue characterization sensors, while allowing the sensors to be kept in place;
**Figs. 7C-7E** exemplify more specifically attachment of the probe with the treatment tool to a hand held device;
**Figs. 8A-8B** show one configuration of a tissue treatment apparatus utilizing the medical device of the present invention;
**Fig. 9** shows a second configuration of a tissue treatment apparatus utilizing the medical device of the present invention;
**Figs. 10A-10B** show an example of a tissue treatment apparatus and a movement mechanism utilizing the medical device of the present invention;
**Fig. 11A** illustrates an example of a tissue treatment apparatus and a manual movement mechanism having another bracket configuration utilizing the medical device of the present invention;
**Fig. 11B** is an enlarged side view of a possible configuration of the bracket element;
**Fig. 12A** illustrates an example of a tissue treatment apparatus and a manual movement mechanism having another bracket configuration utilizing the medical device of the present invention;
**Fig. 12B** is an enlarged side view of another possible configuration of the bracket element;
**Figs. 13A-13B** show an example of a lower part of a tissue treatment apparatus using the medical device of the present invention;
**Figs. 14A-14E** show an example of a tissue treatment apparatus and a movement mechanism having another configuration utilizing the medical device of the present invention;
**Figs. 15A-15C** illustrate an example of a tissue treatment apparatus utilizing the medical device of the present invention; and
**Figs. 16 to 23** illustrate several examples, respectively, for some features of a graphical user interface (GUI) utilized to present information collected by the sensors of the device of the present invention.

### DETAILED DESCRIPTION OF EMBODIMENTS

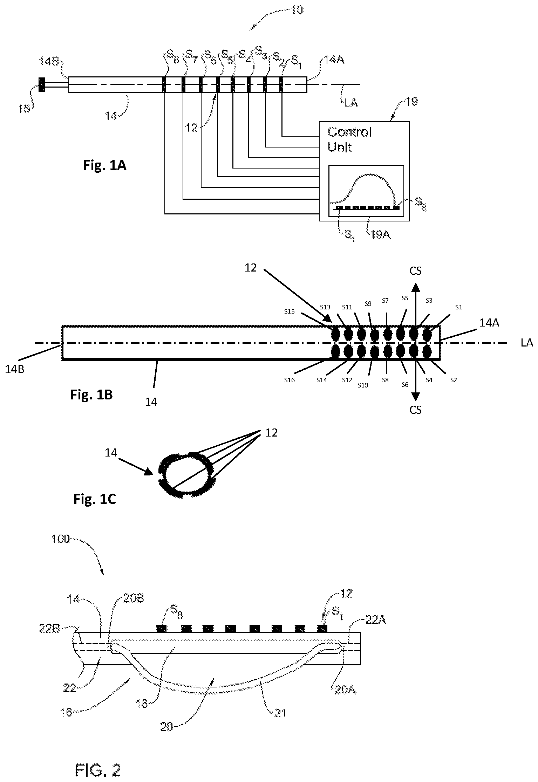

Referring to **Figs. 1A and 1B****,** there is schematically illustrated a medical device, generally designated **10**, according to some embodiments of the invention. The device **10** is configured for use in tissue characterization and treatment, and includes a tissue characterizatio probe **12** carried by an elongated shaft/carrier **14,** which has distal and proximal ends **14A** and **14B,** respectively, and is formed with a control handle portion **15** at its proximal end **14B.** The tissue characterization probe **12** includes a plurality of tissue characterization sensors.

The tissue characterization sensor array may include one or more of optical, radiofrequency (RF), microwave (MW), electrical, magnetic, temperature, elastic, biological, chemical, radioactive-emission, and mechanical sensors of any known type. The construction and operation of the tissue characterization sensor does not form part of the present invention, and therefore need not be specifically described. For example, sensors described in the above indicated patent publications assigned to the assignee of the present application may be used.

The plurality of sensors may be arranged in a one-dimensional array where the sensors are arranged on the surface of the carrier **14** in a spaced-apart relationship only along the longitudinal axis thereof, or may be arranged in a two-dimensional array being in a spaced apart relationship along the longitudinal and lateral axes of the carrier (as may be constituted by example of **Fig. 1B**). In case of a rounded probe (substantially circular cross section), the sensors may be arranged in a two-dimensional array being in a spaced-apart relationship along the carrier's axis **LA** and also along at least a part of the circumference of the carrier, as seen in **Figs. 1B** and **1C****.** At times, the two-dimensional array of the tissue characterization sensors is actually a flexible structure that wraps at least a part of the probe. Such a flexible structure may be implemented as described in WO 2011/016035 which is assigned to the assignee of this application.

In the example of **Fig. 1A**, a plurality of eight sensors **S₁-S₈** is shown, the sensors **S₁-S₈** are arranged in a spaced-apart relationship along a longitudinal axis **LA** of the carrier **14**, and may be arranged in one- or two dimensional array. For example, the sensor array may include, in addition to a group of sensors arranged in one-dimensional array, sensors arranged in a spaced-apart manner along a circumferential region of the carrier. The sensor array gives, in real time, indication about the nature of tissue along the carrier **14.**

**Fig. 1B** exemplifies more specifically another possible configuration of the medical device **10** in which a two-dimensional array of sensors is provided on the carrier **14** extending both along the carrier's axis and its circumference. There are 16 sensors being seen in the figure, but it should be clear that there might be more sensors in the array, for example sensors that cannot be seen in this side view of the probe.

**Fig. 1C** shows the cross sectional view of a carrier **14** taken along the line CS-CS in **Fig. 1B****.** As shown in this example, the sensors are arranged in a spaced-apart relationship along the entire circumference of the carrier. This configuration allows monitoring the surrounding tissue in all directions without the need for turning the probe.

The sensors are spaced from one another a known distance, which may or may not be equal for all the sensors in the array. The known relative locations of the sensors along the carrier's **14** axis and possibly also along the circumference (or lateral axis in general) allows for identifying corresponding locations in a tissue mass when the probe is progressing through the tissue mass (i.e. scans the tissue) based on signals received from the sensors. In this connection, the medical device **10** is associated with an appropriate control system **19** configured for receiving and analyzing the signals generated by the sensors. It should be understood that connection between the sensors and the control unit is shown in the figure schematically, and in case a wired connection is used between the sensors and the control unit, the wires would extend inside the carrier **14** and exit at the proximal end **14B.** The sensors may be connected to the control system using a flexible signal transmission structure as disclosed in the above mentioned WO 2011/016035 assigned to the assignee of the present application.

The control system may be an external system connectable (via wires or wireless signal transmission) to the sensors, or may be a constructional part of the probe itself. The control system, based on the analysis of the received signals, operates for determining a location of the margins of an abnormal tissue region inside the examined tissue mass and generating output data indicative of a dimension of the abnormal tissue region. This allows for consequent treatment of the abnormal tissue region by an appropriate treatment tool.

The control system preferably includes a graphical user interface (GUI) **19A**, and is configured for presenting information related to the signals received from each of the sensors. The signals received from each sensor are indicative of the relative position of that sensor and the tissue property(ies) at said location. The GUI thus presents a map of the tissue property(ies) along the probe propagation axis and preferably also around the probe (i.e. angular resolution at a given location on the probe), obtained without a need to rotate the probe. This information provides the operator with information regarding the tissue type at the locations of the sensors. The information presented on the GUI may assist the operator in analyzing the location and extent of the tissue to be treated. The GUI may be configured as described in co-pending US application Ser. No. 13/676,993.

Generally, the treatment tool may be configured for carrying out one or more of the following: biopsy, cutting, delivering physical treatment, delivering treatment medication, diagnostics. More specifically, the present invention is used for removal of an intact tissue specimen (abnormal tissue) and is therefore described below with respect to this specific but not limiting example.

Preferably, the probe **12** also carries a treatment tool, e.g. a cutting tool. This is implemented by configuring the probe such that the treatment tool can be selectively shiftable between its inoperative position, when it is located substantially entirely inside the carrier **14**, and its operative positions when its one or more excision elements (constituting one or more tissue treating elements) project(s) from the carrier.

In some examples of the invention, the selective projection of the excision element is achieved by using the treatment tool of a kind known in the art, where the excision element projects from the carrier body through an opening made along the body portion while moving with respect to the carrier along an axis inclines with respect to the axis **LA**. In some other examples, the excision element projects from the carrier (e.g. from its distal end) while moving with respect to the carrier substantially along the axis **LA**. Such configurations are also generally known in the art. The treatment tool may be configured with a removed tissue collecting unit, which may or may not be selectively projectable from the probe.

According to the invention, the medical device is configured such that a dimension of the excision element part projecting from the carrier can be controllably adjusted (varied) in accordance with the determined dimension of the abnormal tissue margins, thereby adjusting the excision volume. Preferably, the excision element is configured for both cutting the tissue and collecting the tissue being cut.

The following are some specific but not limiting examples of the configuration of the device of the present invention. The same reference numbers are used for identifying components that are common in all the examples.

**Fig. 2** shows a medical device **100** for treatment of a tissue specimen, e.g. for removal of an intact tissue specimen. The device **100** includes an elongated shaft/carrier **14** on which sensors **S₁-S₈** of a tissue characterization probe **12** are mounted in spaced-apart locations, and a tissue cutting tool (generally, a treatment tool) **16** mounted on the carrier **14**. In this specific not limiting example, the carrier **14** has a hollow body **HP** (of a cylindrical-like shape) and the treatment tool **16** (cutting tool) is insertable into said hollow body **HP**.

Also, in this not-limiting example, the cutting tool **16** has a body portion **22** located inside the body **HP** of the carrier **14**, and an excision element **20** projectable from the body **22** through an opening **18** made in the hollow body **HP** of the carrier **14**. In the figure, the excision element **20** is shown in its operative projecting state. The excision element has a cutting edge **21**, and may be configured to have a cup-like shape when in the projecting state, thereby enabling collection of tissue while being cut during the rotation of the carrier **14** and thus of the excision element **20**.

The excision element **20** extends between its first and second ends **20A** and **20B** which are attached to respective first and second locations on the treatment tool body **22** and spaced-apart along the axis **LA** of the carrier **14**. The treatment tool in this example is configured to enable a controllable change of the dimensions of the excision element **20**. In the present example, this is implemented by making the treatment tool body **22** from two spaced members **22A** and **22B**, where at least one of them is slidable with respect to the other along the carrier axis **LA**. As a result, a distance between the first and second locations, and accordingly the first and second ends **20A** and **20B** of the excision element, changes, thereby enable adjustment of the dimension of the cutting portion **21** projecting through the carrier **14**.

By controlling the location of the excision element distal and proximal ends **20A** and **20B** along the carrier **14**, and thus controlling the excision volume, a user can perform optimal removal of a tissue specimen, for example during a breast biopsy procedure. The entire excision element **20** may be movable along the body **22**. Thus, the excision volume is controlled by user by changing the location of the excision element **20** along the carrier **14** and changing a distance between the distal and proximal ends of the excision element.

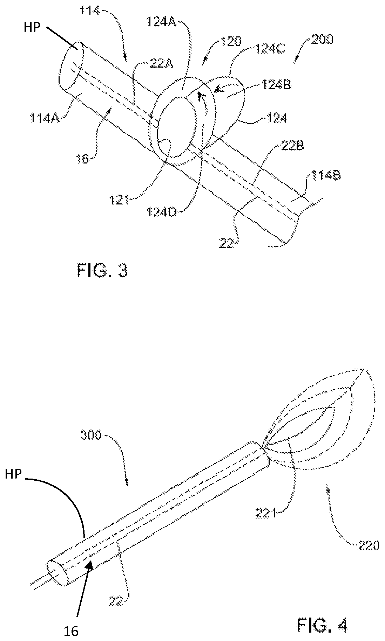

In the above example, the tissue removal procedure is carried while rotating the carrier **14**. Such procedure can be performed while keeping the carrier position and rotating the treatment tool. This is exemplified in **Fig. 3****.** A device **200** includes a carrier **114** formed by two separate parts **114A** and **114B** kept together by a treatment tool **16** inside the carrier **114**. The treatment tool **16** has a body part **22** formed by two spaced-apart members **22A** and **22B**, and an excision element **120** attached thereto by its distal and proximal ends **20A** and **20B**. The excision element **120** has a semi-spherical surface **124** defining a cutting edge **21**. The surface **124** has two arc-like portions **124A** and **124B** movable along the axis **LA** such that when they move towards one another one of the portions **124A** becomes received by the other portion **124B**. Also, the surface **124** has two parts **124C** and **124D** separately movable such that portion **124C** can be received by portion **124C**. These movements allow for altering the excision volume when in the operative projecting state of the excision element **120** and for shifting the element **120** between its operative projecting position and its inoperative position being located inside the carrier **14**. Cutting is implemented while rotating the tool body **22** with respect to the carrier **14**. Also, this configuration allows for collecting the tissue specimen while being cut.

**Fig. 4** shows a medical device **300** according to yet another example of the invention. Here, a treatment tool **16** has a body shaft **22** carrying at its distal end an excision element **220**. The latter may or may not be integral with the body shaft **22**. The excision element has a closed-loop cutting edge **221** which is pre-bent at fabrication so as to deploy from its inoperative closed position when inside the carrier **14** into an open ring-like shape when being projected from the carrier. Attached to the cutting edge **221** is a flexible tissue collecting unit. When the excision element is pushed (by user) out of the carrier **14** through its distal end, it gradually passes through its different operative states being of a spoon-like shape of different dimensions.

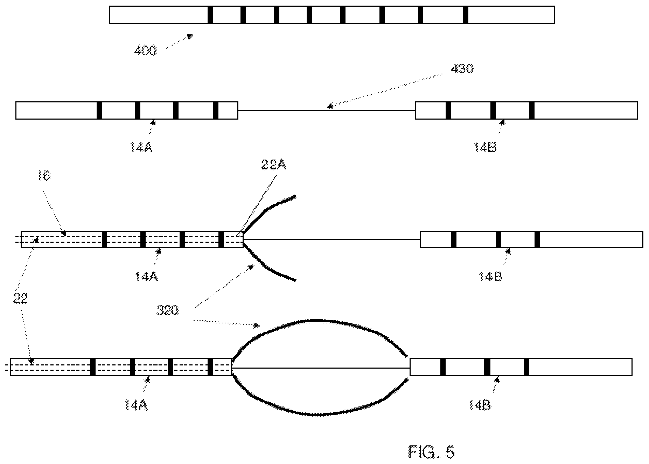

**Fig. 5** shows a medical device **400** according to yet another example of the invention. Here, an elongated shaft (carrier) **14** is separable into two sections **14a** and **14b**, which remain connected to each other by a wire or shaft **430**. A treatment tool **16** located inside the carrier **14** has a body shaft **22** carrying at its distal end **22A** excision elements **320**. The latter may or may not be integral with the body shaft **22**. The treatment tool shaft **22** is advanced inside the shaft **14** until its distal end **22A** reaches the distal end of the section **14A**. Excision elements **220** are then deployed so as to excise an intact tissue portion.

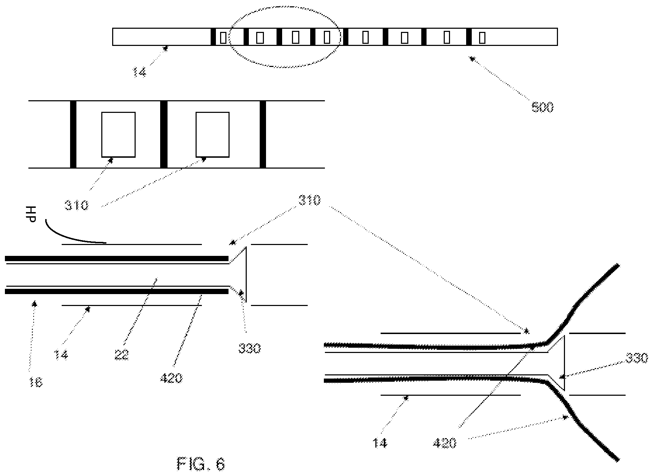

**Fig. 6** shows a medical device **500** according to yet another example of the invention. Here, peripheral slots **310** are provided in the shaft/carrier **14** being interspaced between tissue characterization sensors. At each location along the shaft **14** there may be 2-8 peripheral slots. A treatment tool **16** has a body shaft **22** carrying at its distal end excision elements **420**. The latter may or may not be integral with the body shaft **22**. A number of excision elements **320** corresponds to the number of the slots **310.** The treatment tool **16** is advanced inside the shaft **14** until its distal end is positioned at a specific slot **310** location. The distal end of the treatment tool shaft **22** has a tapered/angled ending portion **330**. This ending portion allows for controlling an angle at which the excision elements **420** is extended from the shaft **14** into tissue. The excision elements **420** are pre-bent so that when extended they close back on the shaft **14**, thus cutting the tissue portion adjacent to the shaft **14**. Additionally, the excision elements **420** may be connected at their ends by wires, or other flexible connection. This connection can be manipulated to induce the contraction of the excision elements **420** towards shaft **14**, to facilitate cutting of the tissue portion adjacent to the shaft **14**.

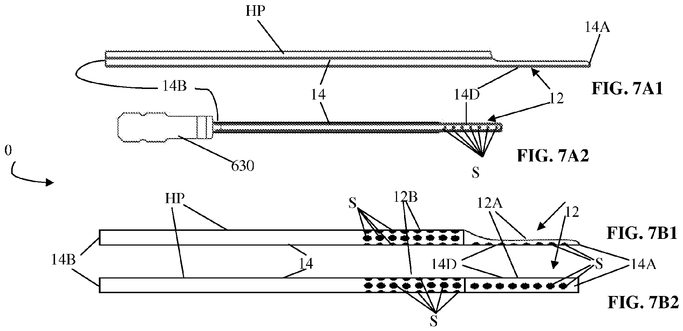

Reference is now made to **Figs. 7A-7E** which show more specifically some exemplified features of the invention. A medical device **600** is shown which is configured for use in tissue characterization and treatment, and includes a tissue characterization probe **12** carried by an elongated shaft/carrier **14** and is configured for carrying a treatment tool. The carrier **14** has distal and proximal ends **14A** and **14B**, and has two integral portions, i.e. a hollow portion **HP** (of a cylindrical-like shape) extending from proximal end **14B** and a distal portion **14D** (e.g. configured as a trough-like portion or an open-cut portion) extending from said hollow portion **HP** towards the distal end **14A**. As will be described below, the treatment tool when attached to / mounted on the medical device is at least partially located inside the hollow portion **HP** and either permanently or selectively (when required) projectable from the hollow portion **HP** such that a part of the treatment tool extends along the distal (trough) portion **14D** to be exposed to the tissue. The hollow portion **HP** may have a thin wall configuration, so it does not add any remarkable thickness to the treatment tool passing through it, which in turn enhances the convenience in using and inserting the device **600** into a subject's body. The tissue characterization probe **12** includes an array of spaced-apart tissue characterization sensors, generally at **S**. The sensors are located on an external side (bottom side) of the trough portion **14D** (there may be additional sensors extending along the hollow portion, as will be described herein below), while the opposite, internal side of the trough portion **14D** serves as a site for locating a portion of the treatment tool while in an operative position thereof.

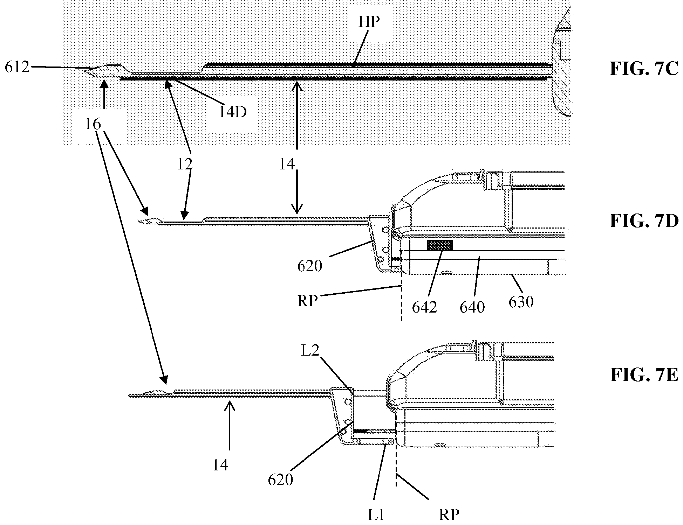

As shown in the example of **Figs. 7A1-7A2****,** the sensors extend along the trough portion **14D**, and may be arranged in a one-dimensional array extending along the longitudinal axis of the carrier **14**. **Figs. 7B1-7B2** show side and bottom views of another possible not limiting example of the configuration of the medical device **600**. The device **600** includes a tissue characterization probe **12** having an elongated shaft/carrier **14** configured for carrying the treatment tool (**610** in Figs. 7C-7E). The carrier **14** has two integral portions, i.e. a hollow portion **HP** (of a cylindrical-like shape) extending from proximal end **14B** of the carrier and a trough-like portion (or an open-cut portion) **14D** extending from said hollow portion towards the distal end **14A** of the carrier. The tissue characterization probe **12** includes two groups of sensors **S**, one group **12A** being located on the trough-like portion **14D** and being arranged for example as a one-dimensional array of spaced-apart tissue characterization sensors, and the other group **12B** of sensors extending along the hollow portion **HP** and including a two-dimensional array of sensors covering the whole or part of the carrier's circumference. The provision of sensors along the hollow portion HP may be very helpful in the monitoring and characterization process may guide the operator and give indication of how large the tissue to be treated by the treatment tool is, thereby saving time and enhancing the patient convenience.

It should be noted that the sensors may be thin, and thus do not protrude too much from the outer surface of the carrier/trough's part **14D**, consequently further enhancing the effectiveness and convenience in using the device inside the subject's body. Such thin and possibly flexible sensor structures are described for example in WO 2011/016035, which is assigned to the assignee of the present application. According to this technique, a sensor unit/structure includes a near field electromagnetic sensor and a flexible signal transmission structure, which are integral with one another by means of one or more common continuous surfaces. The flexible signal transmission structure may be constructed from a first layer including signal connection lines associated with sensor cells of the near field electromagnetic sensor and a second electrically conductive layer electrically coupled to the electrically conductive material of the sensor.

It should be understood that the treatment tool portion may or may not be physically supported by the internal side of the trough portion **14D**. Thus, the treatment tool portion, and accordingly a tissue segment accessed by the treatment tool, can be concurrently aligned with the sensors, enabling the treatment procedure to be carried out without a need to displace the sensing portion of the device. Thus, at least part of the array of sensors is located at the distal part **14D** of the elongated shaft **14**, and this distal part **14D** forms the trough portion of the device which concurrently exposes both the sensors and the treatment tool to the same part/segment of the tissue. It can be appreciated that the device **600** enables immediate treatment or acquisition of very small tissue mass at the exact location of any one of the sensors, during scanning of a tissue mass and without any need for moving the sensors from place as may be required with the above-described device **400** for example.

As shown in **Figs. 7C-7E****,** a treatment tool **16** (e.g. a tissue cutting tool) is carried and housed inside the proximal hollow portion **HP** of the shaft **14** and projects therefrom and extends above the distal, trough portion **14D** of the carrier. The shaft **14** and the treatment tool **16** mounted therein can move forwards and backwards relative to each other. The treatment tool may be formed with a portion thereof configured as an excision element **612** which is in its operative position when it is located above the trough part **14D** or at least partially extends beyond it forwardly into the tissue mass being treated. It should be noted that any suitable treatment tool that conforms to the shape and dimensions of the carrier **14** can be used and the invention is not limited to the cutting tool shown in this specific example, and any other cutting tool as well as any other treatment tools can be used, such as a tool for delivering treatment medication.

The device **600** is typically configured as a hand-held device having a handle portion **630** which is associated with the handle portion (**15** in Fig. 1A) of the carrier **14**, being connectable to or integral with the proximal end **14B** of the carrier **14** (Fig. 7B). Further, the device **600** may include a movement mechanism located at the proximal end **14B** of the carrier **14**, configured and operable to perform relative displacement between the carrier **14** and the treatment tool **16**. The movement mechanism may be manually activated by user and/or may be assisted by a motor unit **640**. The movement mechanism (e.g. the motor unit) may be mounted inside the handle.

The motor unit **640** drives a movement of the carrier/shaft **14** relative to the treatment tool (cutting tool) **610** forwards and backwards as shown in **Figs. 7E** and **7D**, allowing for covering, revealing or repositioning the treatment tool **16** during the treatment process. **Figs. 7D and 7E** exemplify two different relative positions of the carrier and the treatment tool. In this specific but not limiting example, the configuration is such that the carrier **14** moves with respect to the treatment tool **610**. In the example of **Fig. 7D**, the carrier **14** is in its retracted position (retracted towards the handle) exposing a longer portion of the treatment tool to the tissue mass, as compared to the extracted position of the carrier **14** shown in **Fig. 7E**, where a shorter portion of the tissue tool is exposed.

Possibly, the movement mechanism (either manual or driven by motor) includes a registration assembly to define a reference/registration position for the sensors' array with respect to a reference plane, which may be defined by the handle location. This assists in accurate determination of the dimension of an abnormal tissue specimen that is to be treated. The registration assembly may be formed by an L-like shaped bracket which by its one arm **L1** of a given length is movably (telescopically) connected to the handle **630** and by its other arm **L2** is connected to the carrier. Thus, when the arm **L1** moves towards and away from a reference plane **RP** defined by the distal edge of the handle **630** (via a respective guiding mechanism which is not specifically shown) between its retracted and extracted positions with respect to the handle, the carrier **14** becomes correspondingly movable towards and away from the handle. Such configuration assists in monitoring the sensors' repositioning caused by the movement of the carrier which is in turn controlled by the movement of the bracket **620** with respect to the reference plane **RP**. It should be noted, that the reference plane **RP** can be defined at another point along the device, and it is not limited to the shown in **Figs. 7D-****E**, in any case the calculations of distances are adapted to the reference plane chosen.

In some embodiments, the registration assembly of the movement mechanism may include a sensing unit **642** illustrated in **Fig. 7D** (e.g. imaging unit, electro-optic sensor, magnetic sensor) which dynamically identifies the position of the sensors array relative to the reference plane **RP** or to another reference point/position and delivers real-time position data to a control unit that can be located in the handle **630** or outside the medical device or to the control system **19**. Alternatively, the sensing unit may be located on carrier **14** or bracket **620**.

The movement mechanism of the invention may include a locking system, being mechanical, electrical or electro-mechanical, that has the ability to maintain the sensor array **12**, carried by the carrier **14,** at fixed and locked positions relative to the treatment tool **16**. The provision of lock positions enables during an invasive procedure to reduce/overcome the friction forces applied by the body tissues on the medical device. If the movement of the carrier **14** is continuous, the locking system can be actuated at any point along the moving part (e.g. the carrier **14**). Alternatively, if the movement of the carrier **14** occurs in defined steps having known distance (generally in millimeters), the locking system can be activated at those defined steps. For example, the proximal end **14B** of the carrier **14** which is located inside the handle **630** may include a toothed part along the longitudinal axis that engage another toothed part of an adjacent suitable member. The step of the tooth defines the movement steps.

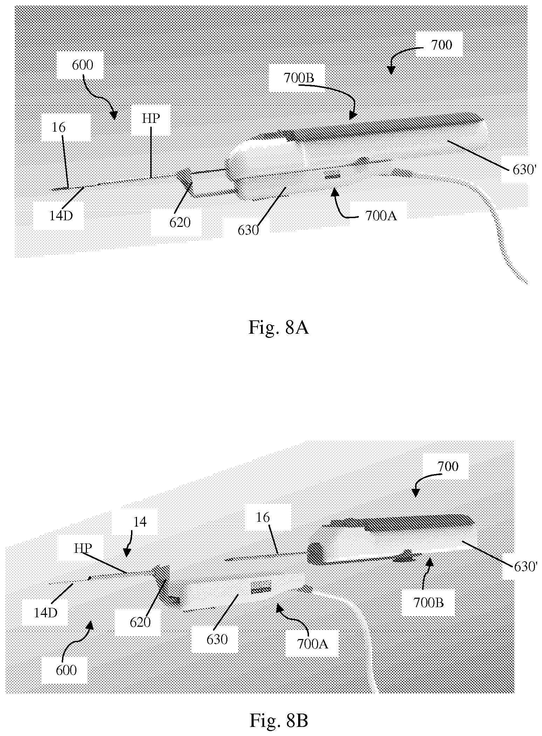

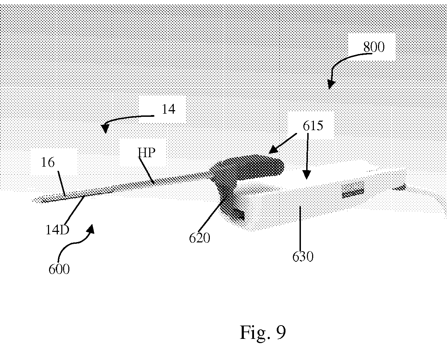

In general, the movement mechanism is configured to provide mechanical support to the relative movement between the carrier **14** and the treatment tool **16**, and at the same time to preserve electrical signal transfer between the sensors and the control unit (transmitter / receiver) which may be located outside the hand-held device. In this specific example of device **600**, there is a rigid connection between the bracket **620** and the carrier **14** which provides for the mechanical support. Inside the handle **630** there is a movement mechanism configured as described above. It should be noted, although not specifically shown that in order to maintain the electrical connectivity between the sensors and a control unit located inside or outside the handle **630**, proper connection arrangement is provided. This may be achieved by using a flexible transmission structure configured as described in the above mentioned WO 2011/016035 which is assigned to the assignee of this application. Alternatively, an electrical connector may include a rigid portion connecting the sensors to a certain location on the part **L1** of the bracket **620**, and a generally flexible cable that connects said location on the part **L1** of the bracket **620** to a location on an electronic panel inside the handle to which the signals from sensors are to be delivered, thus allowing back and forward movement of the carrier **14** while providing electrical signal transfer irrespective of the movement. Reference is now made to **Figs. 8A-8B** **and** **9** showing two different configurations of a tissue treatment apparatus utilizing the medical device of the present invention.

In the example of **Figs. 8A-8B****,** the apparatus, generally designated **700**, is configured as a two-part assembly. As better seen in **Fig. 8B**, the device **600** carrying the tissue characterization probe of the invention is associated with part **700A**, while a treatment tool is carried by the part **700B.** More specifically, the tissue characterization device **600** is associated with a handle portion **630**, the carrier of the tissue characterization probe being appropriately connected to the handle portion **630** e.g. via the registration assembly **620**, and the treatment tool **16** is associated with (mounted on) a handle **630'.** The two parts **700A** and **700B** are configured to engage with one another as shown in **Fig. 8A**, via engagement between the handle portions **630** and **630'** implemented by insertion of the treatment tool **16** into the hollow portion **HP** of the carrier **14** (Fig. 8B); in the engaged position the handles **630** and **630'** are aligned with one another. A suitable locking mechanism may be provided (not shown) securing the engaged position of the handles **630** and **630'** during the apparatus operation thus allowing only the movement of the carrier **14** via the movement of the bracket **620** as described above. It should be noted that the device **600** of the invention may be designed (i.e. the carrier **14**) so as to match the configuration of a suitable/desired treatment tool.

In the example of **Fig. 9**, the tissue treatment apparatus, designated **800**, includes the above-described tissue characterization device **600** which in this specific but not limiting example is mounted on a handle **630** via a bracket **620** associated with an appropriate movement mechanism **615** (which may be manual, automatic, or a combination of both). The bracket **620** may be movable with respect to the reference plane defined by the distal edge of the handle causing a controllable movement of the carrier **14** with respect to the treatment tool **16**.

It should however be noted that for some applications, e.g. in sterile conditions or for disposable usage, the treatment apparatus described may not use any handle and thus may be formed solely by the device **600** (i.e. the carrier **14** configured as exemplified above) and bracket **620**. Alternatively, bracket **620** may be configured so as to enable connection and disconnection of bracket **620** from the handle **630**. This also allows the device **600** (i.e. the carrier **14** configured as exemplified above) and bracket **620** to be disposable and/or sterile one.

The movement mechanism used in the invention may be configured to suit different applications, and its implementation may be influenced by several factors, e.g. the force needed in order to move the carrier relative to the treatment tool during an invasive procedure, the preferred speed of the movement, the accuracy of the movement and others. In the following, more examples of medical apparatuses that contain movement mechanisms having different configurations are presented.

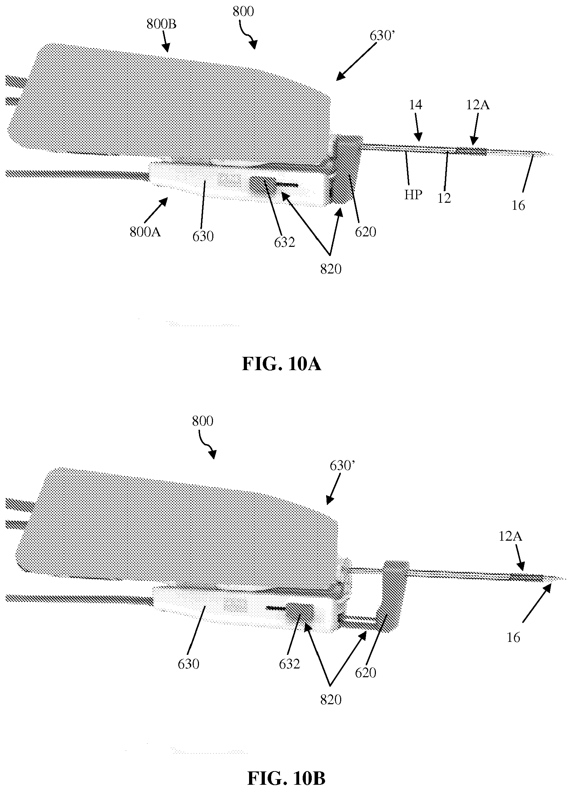

**Figs. 10A-10B** show one example of an apparatus **800** including a specific and non-limiting arrangement of a movement mechanism **820.** In this specific and non-limiting example, the apparatus **800** is configured as a two part-assembly. The device carrying the tissue characterization probe **12** such as the probe in **Fig. 1B** is associated with part **800A**, while a treatment tool **16** is carried by the part **800B.** More specifically, the tissue characterization device is mounted on a handle **630**, e.g. via the bracket **620**, and the treatment tool **610** is mounted on a handle **630'**. The two parts **800A** and **800B** are engaged together and the treatment tool **610** is passed through the probe **12** to form the apparatus **800**.

The probe **12** may include an array of sensors **12A** as described above with respect to **Fig. 1B**. This array of sensors might be configured according to the arrangements disclosed in WO 2011/016035, and may include a signal transmission structure attached to the probe **12**. The structure connects the sensors to a control unit located inside the handle **630** or outside the apparatus.

The apparatus also include a registration assembly. As described above, the registration assembly may be formed by an L-like shaped bracket **620** which by its one arm of a given length is movably (telescopically) connected to the handle **630** and by its other arm is connected to the carrier **14**.

In the specific example described in **Figs. 10A-10B****,** the movement mechanism **820** includes the bracket **620** and a slider **632** configured to generate movement of the shaft **14** relative to the treatment tool (cutting tool) **610** forwards and backwards, the treatment tool **16** between its inoperative position being located substantially entirely inside the hollow portion of the carrier and in its operative position projecting by its treating portion towards outside the carrier. The slider **632** may be located on one side of the handle **630** or on both sides. Moving the slider **632** forwardly as illustrated in **Fig. 10B** causes the bracket **620** and the probe **12** attached to the bracket to move forwardly relative to the treatment tool **16** (and covering the front active part of the treatment tool). Moving the slider **632** backwardly to its back position, as shown in **Fig. 10A**, slides the bracket **620** and the probe **12** back exposing a longer part of the treatment tool's front side, including the excision element **612.** The sliding of the slider **632** may be continuous or by defined pitches that result in known displacements of the carrier 14; increasing the control and the convenient operation of the medical device.

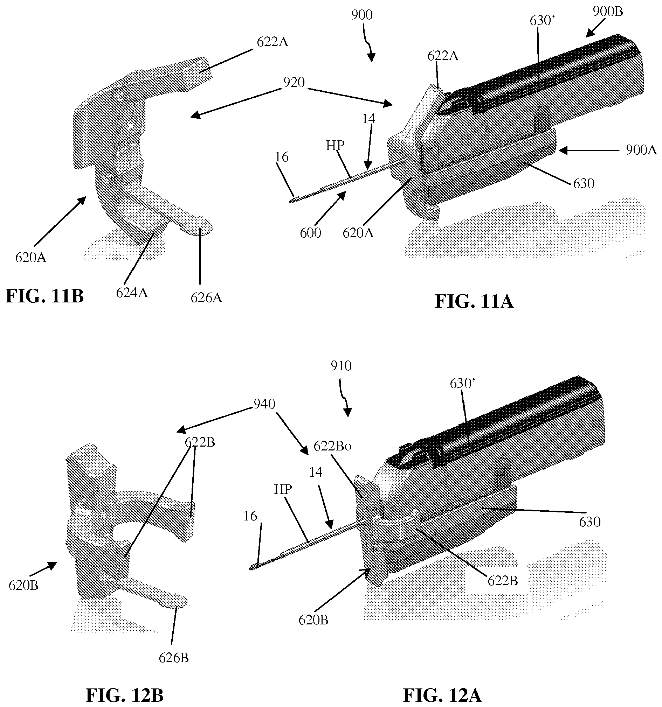

Another not limiting example of a treatment apparatus **900** according to the present invention is shown in **Figs. 11A** and **11B****.** The apparatus is also configured as a two-part assembly. The device **600** carrying the tissue characterization probe of the invention is associated with the lower part **900A,** while a treatment tool is carried by the upper part **900B.** The apparatus **900** has the same features of the apparatus **700** in **Fig. 8A** apart from a different movement mechanism **920**. The movement mechanism **920A** is manual and is associated with a bracket **620A.** The bracket **620A** has an upper region **622A** and a lower region **624A.** During operation, the upper region **622A** or lower region **624A** or both (depending on the apparatus's orientation) can be pushed by the user forwardly, thus moving the hollow part of the carrier **14** that is attached to the bracket **620A** forwardly, thereby covering the front active part of the treatment tool **16**. The user can then move the carrier **14** backwards towards the part **630'** to expose the treatment tool as needed by manually pushing the bracket **620A** backwardly. The languet element **626A** forming the lower part of the L-shape registration assembly enters the part **630** and maintains the balanced straight movement of the carrier **14.** The distal part languet element **626A** serves as an interlock that limits the outward movement of the carrier **14.**

The apparatus **910** may include a movement mechanism **940** associated with another bracket configuration such as the bracket **620B,** as shown in **Figs. 12A** and **12B**. In this specific not limiting example, the movement mechanism **940** is manual and utilizes two lateral arms **622B** which can be pushed forwardly by the user causing the covering the front active part **610** of the treatment tool by the carrier **14.** On the other side, pushing the region **620Bo** of the bracket **620B** retracts the carrier **14** to expose the front active part of the treatment tool. The choice of bracket, **620A** or **620B,** may depend on the effectiveness and/ or convenience of operation which might be affected, *inter alia,* by the part of body being treated.

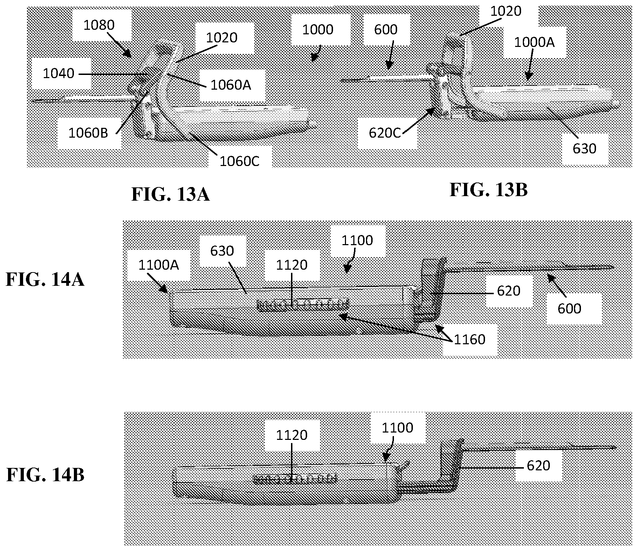

Referring to **Figs. 13A-13B****,** another example of an apparatus **1000** according to the present invention is shown. Here, only the part **630** associated with a lower part **1000A** of the apparatus **1000** and including the tissue characterization device **600** is shown. The second upper part of the assembly associated with the treatment tool **16** is not shown and is similar to anyone of the previously shown apparatus's upper parts such as **700B, 800B** and **900B.** The movement mechanism **1080** (integrated with the part **1000A**) is manual and utilizes a handle **1020,** an intermediate platelet **1040,** connecting hinges **1060A-B-C** and an L-shape bracket **620C.** As shown in the figures, the handle **1020** is connected by its lower side to the lower part **630** of the apparatus via the hinge **1060C,** and to the platelet **1040** via the hinge **1060A.** The platelet is connected through its opposite side to the top side of the bracket **620C** via the hinge **1060B.** **Fig. 13A** shows the handle **1020** in its retracted position, i.e. when the tissue characterization device **600** is kept closer to the apparatus, thus exposing the front active part of the treatment tool. The handle **1020** is configured and operable to be pushed forwardly, as shown in **Fig. 13B****,** causing the relative motion of the platelet **1040** and the bracket **620C** forwardly and covering the front active part of the treatment tool by the carrier **14.** This motion is enabled and governed by the connecting hinges **1060A-B-C.** The special configuration of the movement mechanism **1080** in the apparatus **1000** enables the application of larger forces in comparison to the forces that can be applied in the previously presented system **900**. However, the system **900** may be preferable when a more sensitive and smaller force is needed. Accordingly, when dealing with parts of the body that require the application of such large forces during the treatment procedure, the system **1000** might be used.

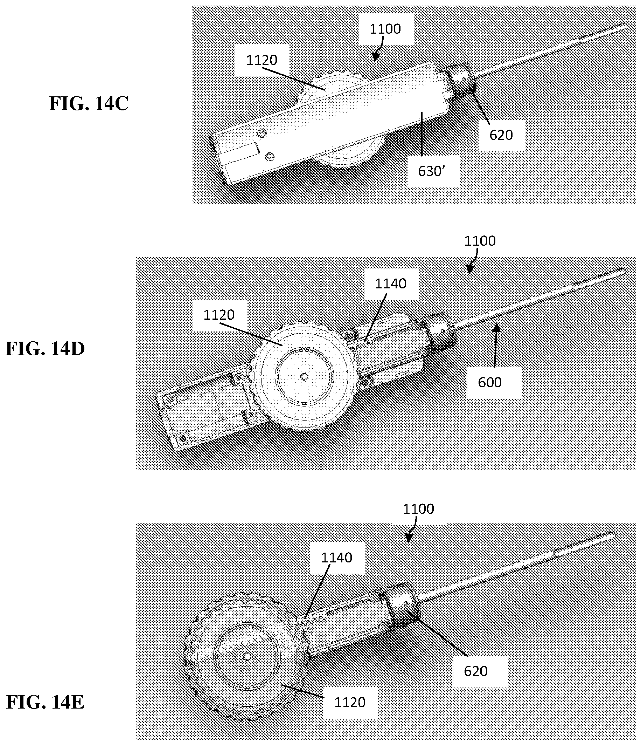

Reference is now made to **Figs. 14A-14E** showing another medical apparatus **1100** utilizing the tissue characterization device **600** of the present invention. The apparatus **1100** is similar to the previous described devices **900** and **1000** and have a two-part assembly configuration, where the upper part is associated with the treatment tool and the lower part is associated with the tissue characterization probe **600**. **Figs. 14A** and **14B** are side views of the retracted and the extracted positions of the bracket **620** (and probe **600**) respectively, **Figs. 14C-14E** are top views of the lower part **1100A** of the apparatus **1100**. Here, the movement mechanism **1160** includes a circular gear **1120** (called "pinion") associated with a linear bar **1140** (called "rack"). As shown, the pinion **1120** and the rack **1140** both have their teeth engaged, so that rotation of the pinion **1120** by the user translates into linear motion of the rack **1140**. The rack **1140** is fixedly attached to the bracket **620**, so when the rack **1140** moves forwardly and backwardly, the bracket **620** and the device **600** will also move forwardly and backwardly.

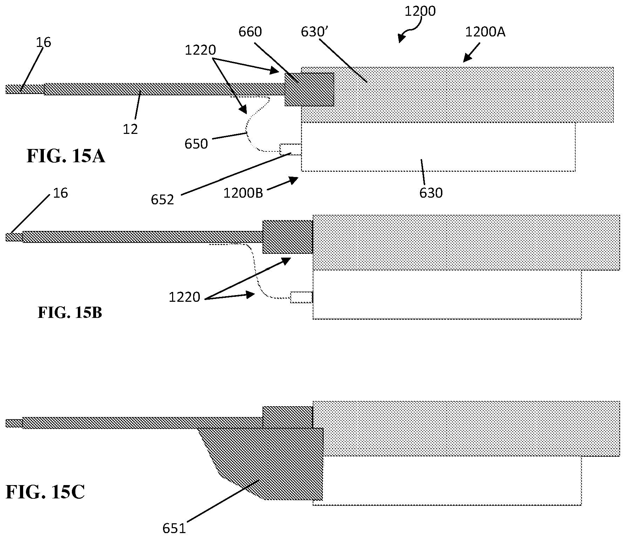

In **Figs. 15A-15C**, a schematic illustration of one more possible embodiment according to the present invention is shown. This embodiment emphasizes the use of the flexible transmission lines described in WO 2011/016035. An apparatus **1200** includes two parts **1200A** (upper) and **1200B** (lower) engaged together. The treatment tool **16** is fixedly attached to the upper part **1200A.** The proximal side of the tissue characterization probe **12** (alternatively device **600**) is securely attached to a base **660** connectable to the part **1200A**, possibly the handle **630'**. The base **660** and the probe **12** (or **600**) surround the treatment tool **16.** The movement mechanism **1220** is configured to enable the forward and backward movement of the base **660** and of the probe **12**. The movement mechanism **1220** utilizes a manual, automatic or hybrid actuation scheme. A sensor array mounted on the probe **12** (not shown) is connected via a flexible transmission line structure **650** that maintains, irrespective of the motion between the probe **12** and handle **630**, the steady electrical transmission of the signals from the sensors to a rigid connector **652** located at the front side of the handle **630**, the signals are then carried from the connector **652** via one or more suitable cables to a control unit located either inside or outside the handle **630**.

In **Fig. 15A****,** the probe **12** is in its retracted position exposing a long portion of the front part of the treatment tool to the tissue mass relatively to the length of the treatment tool. In **Fig. 15B****,** the probe **12** is in its extracted position covering a long portion of the front part of the treatment tool relatively to the length of the treatment tool. The transmission structure **650** is configured and chosen such as to allow this situation by providing it with enough length. In **Fig. 15C** a protective cover **651** placed beside the handle **630** and under the probe **12** is shown. The protective cover **651** protects the transmission line structure.

It should be understood that in all the above-exemplified embodiments the device may be rotated, manually or mechanically, to assist in complete tissue treatment (e.g. cutting and removal). The removal/acquisition of relevant tissue portion may be carried out manually or by using a suctioning mechanism possibly implemented in the apparatus, e.g. in the handle **630'**. The removed tissue is suctioned through the portion **HP** of the carrier **14** and out of the body, to be further treated as desired.

Thus, the present invention provides a novel medical device capable of precisely locating a tissue volume to be treated (removed), and also provides for treating (removing) the tissue by an integral treatment tool.

As described above, the tissue characterization device, and possibly also the treatment tool carried thereby, is connected to the control unit configured for receiving and analyzing the tissue characterizing signals from the sensors and utilizing data indicative of the location of each of the respective sensors for determining the location, and optionally the dimension, of an abnormal tissue specimen, to thereby enable consequent treatment of the abnormal tissue specimen by the treatment tool being carried by the tissue characterization device. The control unit is preferably configured with a graphical user interface (GUI) for presenting information related to the signals received from all the sensors, thereby providing an operator with information regarding the tissue type at the locations of the sensors, and facilitating analysis of a location and extent of the tissue to be treated. The GUI may be configured for presenting an array of measurement locations corresponding to the array of sensors arranged along a measurement axis (axis of the carrier), and presenting a corresponding array of measured data in association with the array of measurement locations. The measured data thus provides an operator with information regarding a measured tissue profile along a tissue mass aligned with the array of sensors. The GUI may be configured for displaying the array of measured data where each data piece is represented by a respective value corresponding to a tissue property being sensed by the respective sensor, and/or a respective figure having a geometry or size corresponding to a tissue property being sensed by the respective sensor, and/or respective figure and value, a geometry or size of the figure and the value corresponding to a tissue property being sensed by the respective sensor.

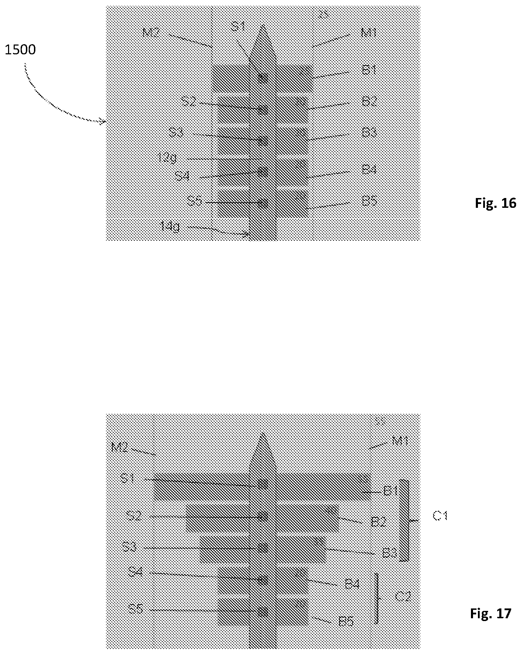

Referring now to **Figs. 16** to **23****,** several examples of a GUI **1500** are shown where information collected by the medical device of the present invention is displayed in real-time.

**Fig. 16** shows the GUI **1500** that graphically illustrates an array **14g** of sensors' positions along an axis **12g** corresponding to that of the carrier in the tissue characterization probe. In this example, five sensors are considered being illustrated by the sensors' positions **S1-S5.** Also displayed is the measured data in the form of a corresponding array of data pieces, which in the present example include values corresponding to certain measured parameter / tissue property. As shown, measured data from sensors **S2-S5** is presented by value "20", while that of sensor **S1** shows higher value "25" of the same measured parameter. Two longitudinal border/limit lines **M1** and **M2** present maximal/minimal values (threshold) or desired values of the specific parameter being displayed. The border lines values may be user set, through the control unit **19**, for each parameter displayed. Moreover, the measured data pieces are presented by bars **B1-B5** where the sizes of the bars correspond to differences in the values. Accordingly, in this example, bars (generally, figures) **B2-B5** are of the same size and bar **B1** is larger. Further, in the present example, the measured data pieces are presented by colorful bars **B1-B5** identically placed on the right and left sides of the sensors (i.e. graphical presentation of the sensors' positions). Each bar displays the value corresponding to the tissue parameter of interest measured by the corresponding sensor, i.e. at the corresponding tissue site.

The two longitudinal border/limit lines **M1** and **M2** may also present dynamically the maximal/minimal value of measured data during a given measurement session (the probe is scanned/moved within the tissue). The maximal/minimal value of measured data is the global value, i.e. taking into account measured data from all the sensors and taking into account the cumulative measured data during the measurement session. In this case, the values (location on GUI) of limit lines **M1** and **M2** are updated in real-time as different measurement values are obtained, as the probe is scanned/moved within the tissue. At a given time, the values (location on GUI) of limit lines **M1** and **M2** present the maximal/minimal value of measured data obtained from the beginning of the measurement session up until the current time. The limit lines values may be reset by the user (or automatically based on pre-specified conditions) during the measurement session, through the control unit **19,** for each parameter displayed. In this case, the values of limit lines **M1** and **M2** present the maximal/minimal value of measured data obtained from the time the values were reset up until the current time.

Thus, a change in the bar geometry or shape or size (height/length) may correspond to a change in the value of the tissue parameter being measured, and the measured value may also be presented numerically on each bar. The values of the parameter, and accordingly the bars lengths (sizes), change in real time whenever the device is moved from point to point inside the tissue (i.e. during scanning). In the present example, all the bars **B1-B5** are of the same color. Generally, the same color may be used to indicate that all the measured data pieces correspond to the same tissue parameter and/or to the condition that the measured parameter values are at the same side with respect to the predefined threshold.

In some embodiments, the bars may exist on one side only of the measurement axis **12g**. In other cases, the same or different measured parameters may be displayed simultaneously at both sides of the measurement axis **12g**.

In order to simplify the illustration and understanding, all the figures **16** to **23** exemplify measurements taken by the same number of 5 sensors arranged along the measurement axis, and accordingly the same reference numbers are used throughout all these figures. Every bar / measured data piece is associated with one sensor location. At times, more than one bar may be displayed in association with the same sensor, in which case the bars are usually in different colors, to provide information about more than one parameter / condition simultaneously.

As described above, the sensors may be configured to measure several tissue parameters, such as conductivity, permittivity, impedance, optical property, mechanical property, temperature and others. The bars **B1-B5** and/or values on the display correspond to the strength or intensity of the measured parameter. The values usually correspond to properly normalized parameter and are thus non-dimensional (relative units).

As also described above and will be more specifically exemplified in the figures below, the bars may be presented in different colors to indicate either special (exceptional) values of the same parameter, or different parameters presented simultaneously at different sensor/bar couples. The bar color and/or size together with the border lines may be exploited to indicate a range of values. Further, the GUI **1500** may include an indication about the mean value of some or all of the measured parameters by lighting up a specific region on the GUI.

**Fig. 17** illustrates an example in which bars **B1-B3** have different colors and sizes: bars **B1-B3** are of first color **C1** (Orange) and different sizes corresponding to values 55, 40 and 35 respectively, while **B4** and **B5** are of the same size each with the value 20 and are displayed in second color **C2** (Blue). In this example, the values of border lines **M1** and **M2,** indicating a maximal value of the measured parameter, are located at value 55 (which coincides with the value of bar **B1**), while a range of values corresponding to the measured parameter indicative of abnormal tissue property is considered to be between 30-55. Thus, the size and color of bars **B1-B3** provide immediate indication of the abnormal condition of the tissue. It should, however, be understood that a different color of bars may be used to indicate measurements of another tissue property.

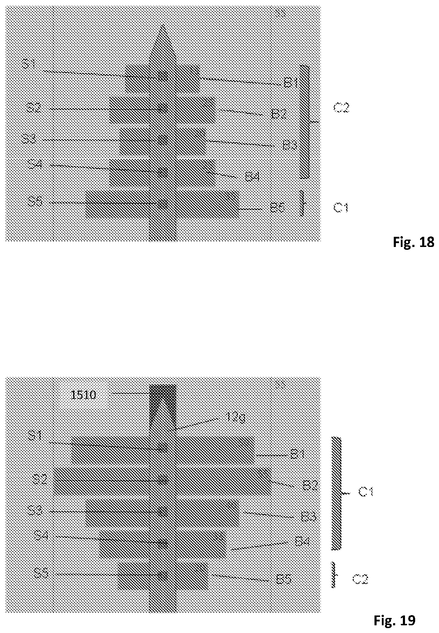

Another similar example, where the bar color and size together with the border lines are used to indicate a range of values, is illustrated in **Fig. 18**. If the measured parameter at a specific location is higher than a minimal value, the corresponding bar has a different color, while the border lines indicate the maximal value of the range, or the maximal value obtained during the measurement session up until the current time. In this example, the minimal value is set to be 30, and the maximal one is at 55. As shown in the figure, each of the measured locations (sensors' position) **S1** to **S4** shows a different value lower than a minimum one, and accordingly the respective bars **B1** to **B4** are displayed with different sizes but all of the same second color **C2** (Blue). The measured data piece at the sensor's position **S5** has a value higher than minimum, and its respective bar **B5** has a larger size and a first color **C1** (Orange). On the other side, all the bars show values lower than the maximum value 55 presented by the border lines **M1** and **M2.**

As exemplified in **Fig. 19**, if the number of measured data pieces (bars) corresponding to high/low values with respect to a predefined threshold and/or the mean value of the measured data pieces exceeds or does not exceed a certain value, then a specific indicator (e.g. color indicator) **1510** is displayed indicating the condition that the tissue property at a specific tissue site is higher/lower than a predetermined threshold predefined for the specific parameter. The use of such additional indicator provides additional indications to the operator in real-time. The specific color of the indicator (red color in the present example) may indicate a "problematic" value whether being higher or lower than a normal one, and another color of the indicator (or absence of any such indicator) may indicate that the measured parameter is in the safe range. **Fig. 19** may exemplify the condition that the mean value of the measured data pieces is higher than a maximal threshold, even though not all the corresponding data pieces have reached the maximum 55 represented by the border lines. The value of each measured data piece concerns localized information while sometimes the operator is interested in more global information relating to a tissue site and not only to a concrete point in the tissue.

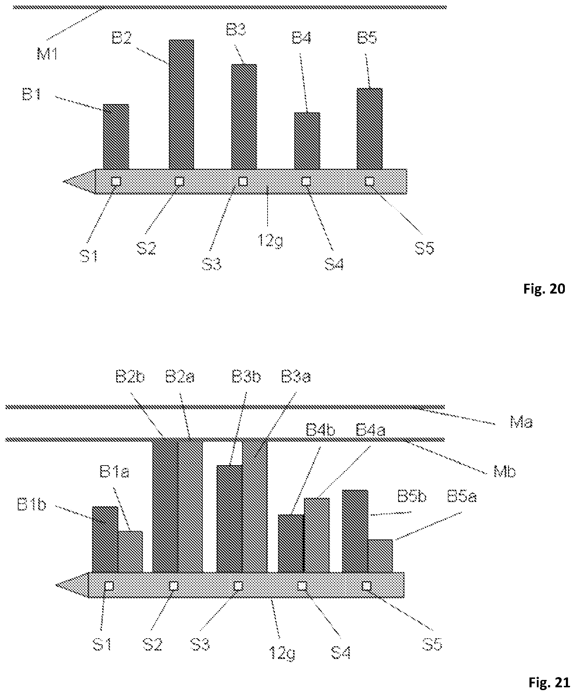

As exemplified in **Fig. 20**, the GUI **1500** may be designed to present a profile of the measured data pieces with respect to a predefined threshold **M1** only by the sizes of the bars, and the bars may be only at one side of the measurement axis **12**.

Further, the GUI **1500** may be used to display more than one parameter at the same time for each sensor as can be seen in the example of **Fig. 21. Fig. 21** shows, for example, a pair of bars associated with each one of the sensors' positions **S1-S5.** Each bar indicates the value of relevant parameter chosen by the operator. The two bars in the pair may be distinguished by their colors, for example first color (Orange) and second color (Blue). Also, the parameters have their corresponding thresholds presented by respective border lines **Ma** and **Mb.**

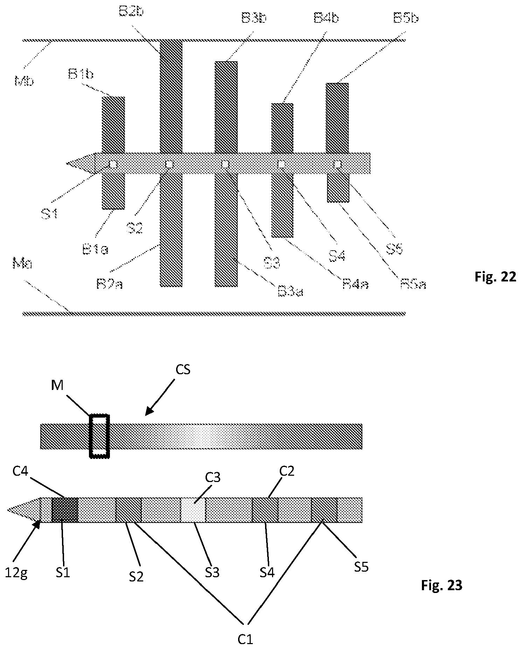

The GUI of the present invention may be configured for displaying different parameters simultaneously at both sides of the measurement axis **12g**. This is illustrated in **Fig. 22**. It should be noted that the GUI may also be configured to combine the feature of **Fig. 21** with the feature of **Fig. 22** to give information about 4 parameters at once, two at each side of the measurement axis **12g**. In the same way, the GUI may display any useful number of parameters concurrently, whether odd or even number. The only limitations on the number of parameters displayed simultaneously would be the overall size/area of the interface and the convenience and readability of the displayed information.

The GUI of the present invention may be configured for displaying the different measured data in the form of different colors, according to a predetermined color scheme, without assigning a bar presentation. This is illustrated in **Fig. 23**. In this case, the color scheme **CS** may be presented on the GUI as well. The limit value(s) are captured by an indicator **M** on the color scheme. As shown in the figure, the measurement locations / sensors' positions are presented by GUI as an array of spaced-apart colored regions, where different colors correspond to different values of the measured data. The sensors **S2** and **S5** show the same measured data, different from that of the other sensors **S1**, **S3** and **S4**, and accordingly, the corresponding regions/locations on the display are presented by the first color **C1** (Orange) different from other colors. The sensors **S1, S3** and **S4** show different measured data and accordingly the corresponding locations on the display are colored by different colors **C4**, **C3** and **C2** (red, yellow and blue respectively). The displayed colored sensing positions together with the color scheme **CS** with the indicator **M** provide immediate information of the profile of tissue property along the measurement axis showing that at sensing positions **S1, S2** and **S5** the tissue property is close to the threshold.

The graphical presentation of the information from the measured data, as presented in the examples related to figures 16-23, can also be overlaid/merged with imaging data information obtained from other modalities. For example, the information may be merged with Ultrasound, X-ray, or PET (Positron Emission Tomography) image used to guide the probe to specific regions of the tissue. The visibility of the probe on the other imaging modalities is used to identify the location of the sensors on the merged image.

Thus, the present invention provides a novel medical device capable of precisely locating a tissue portion to be treated (removed), and also provides for treating (removing) the tissue by an integral medical device. The invention also provides for displaying the measured data in a manner providing immediate and clear indication of the profile of the tissue parameter(s) being measured.

## Claims

1. A medical device for use in tissue characterization and treatment, the device comprising
a tissue characterization probe comprising an elongated carrier having a longitudinal axis and proximal and distal ends, and carrying an array of tissue characterization sensors, each sensor being configured and operable for generating a tissue characterization signal corresponding to a tissue mass at a location of the sensor, said tissue characterization sensors being arranged in a spaced-apart relationship along at least said longitudinal axis and within at least a distal portion of said elongated carrier with known distances between them, such that during progression of the probe through a tissue mass each of the sensors generates tissue characterization signals from successive locations thereof within the tissue mass thereby enabling to locate and determine a dimension of a tissue specimen inside said tissue mass based on the characterization signals from the sensors in the array;
wherein
said elongated carrier has two integral portions including a proximal portion and said distal portion extending successively along said longitudinal axis from said proximal end to said distal portion of the elongated carrier, said proximal portion having a cylindrical-like hollow shape, said distal portion having a trough-like shape being open-cut both along said longitudinal axis and along at least part of a circumference of the distal portion,
said elongated carrier being thereby configured for passing a treatment tool through the hollow proximal portion and enabling at least a distal part of the treatment tool to project from the hollow proximal portion and extend forwardly on the longitudinal axis along the distal portion and forwardly beyond said distal end of the elongated carrier,
the device thereby enabling consequent treatment by the distal part of the treatment tool of a tissue specimen at exact location of any sensor of said sensors arranged on said distal portion while the distal part of the treatment tool and the tissue specimen both being concurrently aligned with said sensor and while keeping the
elongated carrier in place during the tissue treatment, and of a tissue specimen located beyond said distal end of the elongated carrier.

2. The device according to Claim 1, wherein the array of tissue characterization sensors further comprises sensors arranged in a spaced-apart relationship along a circumferential region of the elongated carrier within at least a part of said proximal portion.

3. The device according to any one of the preceding Claims, comprising a handle portion connectable to or integral with the proximal end of the carrier.

4. The device according to Claim 3, wherein the handle portion is configured for engaging with a handle portion of a predetermined treatment tool when the treatment tool is being inserted into the hollow portion of the carrier.

5. The device according to any one of the preceding Claims, comprising a movement mechanism located at the proximal end of the carrier, said movement mechanism being configured and operable to perform relative displacement between the carrier and a treatment tool being carried thereby.

6. The device according to Claim 5, wherein the movement mechanism is located inside a handle portion which is connectable to or integral with the proximal end of the carrier.

7. The device according to Claim 5 or 6, wherein the movement mechanism is **characterized by** at least one of the following:
(i) the movement mechanism comprises a registration assembly to define a reference position for the array of the tissue characterization sensors with respect to a certain reference plane defined by the device, thereby enabling to monitor repositioning of the tissue characterization sensors caused by movement of the carrier with respect to the reference plane;
(ii) the movement mechanism comprises a locking system configured to maintaining said sensor array at fixed and locked positions;
(iii) the movement mechanism comprises a slider configured to provide said relative displacement enabling selective displacement of a treatment tool, being carried by the carrier, between inoperative and operative positions;
(iv) the movement mechanism is configured to convert a rotational movement into the linear relative displacement between the carrier and a treatment tool being carried thereby;
(v) the movement mechanism comprises rack and pinion configuration.

8. The device according to Claim 7, wherein the movement mechanism comprises the registration assembly, said registration assembly comprising at least one of the following: (a) a sensing unit configured and operable to identify a position of the sensors array relatively to said reference plane and to generate data indicative thereof; (b) an L-like shaped bracket which by its one arm of a given length is movably connected to the handle portion and by its other arm is connected to the carrier, the reference plane being defined by a distal edge of the handle portion, thereby enabling to monitor repositioning of the tissue characterization sensors caused by movement of the bracket with respect to the reference plane.

9. The device according to any one of the preceding Claims, being configured as a two-part assembly comprising a first assembly and a second assembly, the first and second assemblies being removably attachable to one another, the two-part assembly having one of the following configurations: (i) said first assembly carries the tissue characterization probe and the second assembly is configured for carrying a treatment tool; (ii) either one of said first and second assemblies is configured for carrying the tissue characterization probe and a treatment tool.

10. The device of any one of the preceding Claims, comprising a control unit configured for receiving and analyzing the tissue characterizing signals from the sensors and utilizing data indicative of the location of each of the respective sensors for determining a location of an abnormal tissue specimen, thereby enabling consequent treatment of the abnormal tissue specimen by a treatment tool being carried by the carrier.

11. The device according to Claim 10, wherein the control unit has at least one of the following configurations: comprises a graphical user interface configured for presenting information related to the signals received from all the sensors, thereby providing an operator with information regarding the tissue type at the locations of the sensors, and facilitating analysis of a location and extent of the tissue to be treated; and is configured for receiving from one or more imaging modalities imaging data of the tissue characterization probe position with respect to tissue being measured, and merging the imaging data with the measured data, to thereby identify locations of the sensors on a merged image.

12. The device of Claim 11, wherein the control unit comprises said graphical user interface, the graphical user interface being configured for presenting an array of measurement locations corresponding to the array of said sensors arranged along said at least axis being a measurement axis, and presenting a corresponding array of measured data in association with the array of measurement locations, said array of measured data being indicative of the signals received from the array of the sensors respectively, thereby providing an operator with information regarding a measured tissue profile along a tissue mass aligned with the array of sensors.

13. The device of Claim 12, wherein said graphical user interface is configured for displaying said array of measurement locations in the form of the array of measured data each being represented by one of the following: (1) a respective value corresponding to a tissue property being sensed by the respective sensor, an array of the values defining the profile of the measured tissue property within a region aligned with the array of sensors; (2) a respective figure having a geometry or size corresponding to a tissue property being sensed by the respective sensor, an array of the figures defining the profile of the measured tissue property within a region aligned with the array of sensors; (3) respective figure and value, a geometry or size of the figure and the value corresponding to a tissue property being sensed by the respective sensor, an array of the figures and values defining the profile of the measured tissue property within a region aligned with the array of sensors.

14. The device of Claim 13, wherein said graphical user interface has at least one of the following configurations: is configured for displaying said figures as colored figures, such that the figures corresponding to the same measured data have the same color and figures corresponding to different measured data have different colors; is configured for displaying data indicative of a threshold value for the tissue property being measured; and is configured for displaying said array of measurement locations in the form of the array of measured data each being represented by a colored region, such that the regions corresponding to the same measured data have the same color and regions corresponding to different measured data have different colors.

## Patentansprüche

1. Medizinische Vorrichtung zur Verwendung bei der Gewebecharakterisierung und -behandlung, wobei die Vorrichtung umfasst:
eine Gewebecharakterisierungssonde, die einen länglichen Träger umfasst, der eine Längsachse und proximale und distale Enden aufweist, und die eine Anordnung von Gewebecharakterisierungssensoren trägt, wobei jeder Sensor so konfiguriert und betreibbar ist, dass er ein Gewebecharakterisierungssignal erzeugt, das einer Gewebemasse an einem Standort des Sensors entspricht, wobei die Gewebecharakterisierungssensoren entlang zumindest der Längsachse und innerhalb zumindest eines distalen Abschnitts des länglichen Trägers mit bekannten Abständen dazwischen zueinander beabstandet sind, so dass jeder der Sensoren während des Fortbewegens der Sonde durch eine Gewebemasse Gewebecharakterisierungssignale von aufeinanderfolgenden Standorten dieser innerhalb der Gewebemasse erzeugt, wodurch es möglich ist, eine Gewebeprobe innerhalb der Gewebemasse auf Basis der Charakterisierungssignale der Sensoren in der Anordnung zu lokalisieren und deren Dimension zu bestimmen;
wobei:
der längliche Träger zwei integrale Abschnitte aufweist, die einen proximalen Abschnitt und den distalen Abschnitt beinhalten, die sich aufeinanderfolgend entlang der Längsachse vom proximalen Ende zum distalen Endabschnitt des länglichen Trägers erstrecken, wobei der proximale Abschnitt eine zylinderähnliche Hohlform aufweist, wobei der distale Abschnitt eine trogähnliche Form aufweist, die sowohl entlang der Längsachse als auch entlang zumindest eines Teils eines Umfangs des distalen Abschnitts aufgeschnitten ist,
der längliche Träger dadurch so konfiguriert ist, dass er ein Behandlungswerkzeug durch den hohlen proximalen Abschnitt leitet und zumindest einem distalen Teil des Behandlungswerkzeugs ermöglicht, vom hohlen proximalen Abschnitt vorzustehen und sich auf der Längsachse entlang des distalen Abschnitts nach vorne zu erstrecken und sich nach vorne über das distale Ende des länglichen Trägers hinaus zu erstrecken,
wobei die Vorrichtung somit eine daraus folgende Behandlung durch den distalen Teil des Behandlungswerkzeugs einer Gewebeprobe an einem exakten Standort eines beliebigen Sensors der Sensoren ermöglicht, die auf dem distalen Abschnitt angeordnet sind, während der distale Teil des Behandlungswerkzeugs und die Gewebeprobe jeweils konkurrierend mit dem Sensor ausgerichtet sind und während der längliche Träger während der Gewebebehandlung an seiner Stelle gehalten wird, und einer Gewebeprobe, die sich jenseits des distalen Endes des länglichen Trägers befindet.

2. Vorrichtung nach Anspruch 1, wobei die Anordnung von Gewebecharakterisierungssensoren ferner Sensoren umfasst, die entlang einer Umfangsregion des länglichen Trägers innerhalb zumindest eines Teils des proximalen Abschnitts zueinander beabstandet angeordnet sind.

3. Vorrichtung nach einem der vorstehenden Ansprüche, die einen Griffabschnitt umfasst, der mit dem proximalen Ende des Trägers verbindbar oder damit integral ausgebildet ist.

4. Vorrichtung nach Anspruch 3, wobei der Griffabschnitt so konfiguriert ist, dass er mit einem Griffabschnitt eines vordefinierten Behandlungswerkzeugs in Eingriff gelangt, wenn das Behandlungswerkzeug in den hohlen Abschnitt des Trägers eingeführt wird.

5. Vorrichtung nach einem der vorstehenden Ansprüche, die einen Bewegungsmechanismus umfasst, der sich am proximalen Ende des Trägers befindet, wobei der Bewegungsmechanismus so konfiguriert und betreibbar ist, dass er eine relative Verschiebung zwischen dem Träger und einem dadurch getragenen Behandlungswerkzeug durchführt.

6. Vorrichtung nach Anspruch 5, wobei sich der Bewegungsmechanismus innerhalb eines Griffabschnitts befindet, der mit dem proximalen Ende des Trägers verbindbar oder damit integral ausgebildet ist.

7. Vorrichtung nach Anspruch 5 oder 6, wobei der Bewegungsmechanismus durch zumindest eines des Folgenden gekennzeichnet ist:
(i) der Bewegungsmechanismus umfasst eine Registrierungsbaugruppe, um eine Referenzposition für die Anordnung von Gewebecharakterisierungssensoren in Bezug auf eine gewisse Referenzebene zu definieren, die von der Vorrichtung definiert wird, wodurch es möglich ist, eine Neupositionierung der Gewebecharakterisierungssensoren, die durch eine Bewegung des Trägers verursacht wird, in Bezug auf die Referenzebene zu überwachen;
(ii) der Bewegungsmechanismus umfasst ein Verriegelungssystem, das so konfiguriert ist, dass es die Sensoranordnung in fixen und verriegelten Positionen hält;
(iii) der Bewegungsmechanismus umfasst einen Schieber, der so konfiguriert ist, dass er die relative Verschiebung bereitstellt, wodurch eine selektive Verschiebung eines vom Träger getragenen Behandlungswerkzeugs zwischen Außer-Betrieb- und In-Betrieb-Positionen ermöglicht wird;
(iv) der Bewegungsmechanismus so konfiguriert ist, dass er eine Drehbewegung in die lineare relative Verschiebung zwischen dem Träger und einem dadurch getragenen Behandlungswerkzeug umwandelt;
(v) der Bewegungsmechanismus umfasst eine Zahnstangenkonfiguration.

8. Vorrichtung nach Anspruch 7, wobei der Bewegungsmechanismus die Registrierungsbaugruppe umfasst, wobei die Registrierungsbaugruppe zumindest eines des Folgenden umfasst: (a) eine Erfassungseinheit, die so konfiguriert und betreibbar ist, dass sie eine Position der Sensoranordnung in Bezug auf die Referenzebene identifiziert und dafür indikative Daten generiert; (b) eine L-ähnlich geformte Halterung, die über ihren einen Arm einer bestimmten Länge mit dem Griffabschnitt beweglich verbunden ist und die über ihren anderen Arm mit dem Träger verbunden ist, wobei die Referenzebene durch einen distalen Rand des Griffabschnitts definiert wird, wodurch es möglich ist, eine Neupositionierung der Gewebecharakterisierungssensoren, die durch eine Bewegung der Halterung verursacht wird, in Bezug auf die Referenzebene zu überwachen.

9. Vorrichtung nach einem der vorstehenden Ansprüche, die als zweiteilige Baugruppe konfiguriert ist, die eine erste Baugruppe und eine zweite Baugruppe umfasst, wobei die erste und die zweite Baugruppe lösbar aneinander befestigbar sind, wobei die zweiteilige Baugruppe eine der folgenden Konfigurationen aufweist: (i) die erste Baugruppe trägt die Gewebecharakterisierungssonde und die zweite Baugruppe ist so konfiguriert, dass sie ein Behandlungswerkzeug trägt; (ii) die erste und die zweite Baugruppe sind jeweils so konfiguriert, dass sie die Gewebecharakterisierungssonde und ein Behandlungswerkzeug tragen.

10. Vorrichtung nach einem der vorstehenden Ansprüche, die eine Steuereinheit umfasst, die so konfiguriert ist, dass sie die Gewebecharakterisierungssignale der Sensoren empfängt und analysiert und Daten, die für den Standort jedes der jeweiligen Sensoren indikativ sind, zum Bestimmen eines Standorts einer anomalen Gewebeprobe verwendet, wodurch eine daraus folgende Behandlung der anomalen Gewebeprobe durch ein vom Träger getragenes Behandlungswerkzeug möglich wird.

11. Vorrichtung nach Anspruch 10, wobei die Steuereinheit zumindest eine der folgenden Konfigurationen aufweist: Sie umfasst eine graphische Benutzeroberfläche, die so konfiguriert ist, dass sie Informationen zu den von allen Sensoren empfangenen Signalen präsentiert, wodurch ein Benutzer Informationen zum Gewebetyp an den Standorten der Sensoren erhält und eine Analyse eines Standorts und eines Ausmaßes des zu behandelnden Gewebes erleichtert wird; und sie ist so konfiguriert, dass sie Bildgebungsdaten zur Gewebecharakterisierungssondenposition in Bezug auf gemessenes Gewebe von einer oder mehreren Bildgebungsmodalitäten empfängt und die Bildgebungsdaten mit den gemessenen Daten zusammenführt, um Standorte der Sensoren auf einem zusammengeführten Bild zu identifizieren.

12. Vorrichtung nach Anspruch 11, wobei die Steuereinheit die graphische Benutzeroberfläche umfasst, wobei die graphische Benutzeroberfläche so konfiguriert ist, dass sie eine Anordnung von Messstandorten präsentiert, die der Anordnung der Sensoren entsprechen, die entlang zumindest der Achse angeordnet sind, die eine Messachse ist, und dass sie eine entsprechende Anordnung von gemessenen Daten in Assoziation mit der Anordnung von Messstandorten präsentiert, wobei die Anordnung von gemessenen Daten für die Signale indikativ ist, die von der Anordnung von Sensoren jeweils empfangen werden, wodurch ein Benutzer Informationen zu einem gemessenen Gewebeprofil entlang einer Gewebemasse erhält, die mit der Anordnung von Sensoren ausgerichtet ist.

13. Vorrichtung nach Anspruch 12, wobei die graphische Benutzeroberfläche so konfiguriert ist, dass sie die Anordnung von Messstandorten in Form der Anordnung von gemessenen Daten anzeigt, die jeweils durch eines des Folgenden dargestellt werden: (1) einen jeweiligen Wert, der einer Gewebeeigenschaft entspricht, die vom jeweiligen Sensor erfasst wird, wobei eine Anordnung der Werte das Profil der gemessenen Gewebeeigenschaft innerhalb einer Region definiert, die mit der Anordnung von Sensoren ausgerichtet ist; (2) eine jeweilige Figur mit einer Geometrie oder Größe, die einer vom jeweiligen Sensor erfassten Gewebeeigenschaft entspricht, wobei eine Anordnung der Figuren das Profil der gemessenen Gewebeeigenschaft innerhalb einer Region definiert, die mit der Anordnung von Sensoren ausgerichtet ist; (3) eine jeweilige Figur und einen jeweiligen Wert, wobei eine Geometrie oder Größe der Figur und des Werts einer vom jeweiligen Sensor erfassten Gewebeeigenschaft entsprechen, wobei eine Anordnung der Figuren und Werte das Profil der gemessenen Gewebeeigenschaften innerhalb einer Region definieren, die mit der Anordnung von Sensoren ausgerichtet ist.

14. Vorrichtung nach Anspruch 13, wobei die graphische Benutzeroberfläche zumindest eine der folgenden Konfigurationen aufweist: Sie ist so konfiguriert, dass sie die Figuren als farbige Figuren darstellt, so dass die den gleichen gemessenen Daten entsprechenden Figuren die gleiche Farbe aufweisen und unterschiedlichen gemessenen Daten entsprechende Figuren unterschiedliche Farben aufweisen; sie ist so konfiguriert, dass sie Daten anzeigt, die für einen Schwellenwert für die gemessene Gewebeeigenschaft indikativ sind; und sie ist so konfiguriert, dass sie die Anordnung von Messstandorten in Form der Anordnung von gemessenen Daten anzeigt, die jeweils durch eine farbige Region dargestellt werden, so dass die Regionen, die den gleichen gemessenen Daten entsprechen, die gleiche Farbe aufweisen und Regionen, die unterschiedlichen gemessenen Daten entsprechen, unterschiedliche Farben aufweisen.

## Revendications

1. Dispositif médical pour une utilisation dans la caractérisation et le traitement de tissu, le dispositif comprenant :
une sonde de caractérisation de tissu comprenant un support allongé ayant un axe longitudinal et des extrémités proximale et distale, et portant un réseau de capteurs de caractérisation de tissu, chaque capteur étant configuré et actionnable pour générer un signal de caractérisation de tissu correspondant à une masse de tissu à un emplacement du capteur, lesdits capteurs de caractérisation de tissu étant agencés dans une relation espacée le long au moins dudit axe longitudinal et à l'intérieur d'au moins une partie distale dudit support allongé avec des distances connues entre eux, de telle sorte que, pendant la progression de la sonde à travers une masse de tissu, chacun des capteurs génère des signaux de caractérisation de tissu à partir d'emplacements successifs de celui-ci à l'intérieur de la masse de tissu, permettant ainsi de localiser et de déterminer une dimension d'un échantillon de tissu dans ladite masse de tissu sur la base des signaux de caractérisation provenant des capteurs dans le réseau ;
ledit support allongé ayant deux parties d'un seul tenant comprenant une partie proximale et ladite partie distale s'étendant successivement le long dudit axe longitudinal de ladite extrémité proximale à ladite partie distale du support allongé, ladite partie proximale ayant une forme creuse de type cylindrique, ladite partie distale ayant une forme de type creux qui est ouverte à la fois le long dudit axe longitudinal et le long d'au moins une partie d'une circonférence de la partie distale,
ledit support allongé étant ainsi configuré pour faire passer un outil de traitement à travers la partie proximale creuse et permettre à au moins une partie distale de l'outil de traitement de faire saillie à partir de la partie proximale creuse et de s'étendre vers l'avant sur l'axe longitudinal le long de la partie distale et vers l'avant au-delà de ladite extrémité distale du support allongé,
le dispositif permettant ainsi un traitement conséquent, par la partie distale de l'outil de traitement, d'un échantillon de tissu à un emplacement exact de n'importe quel capteur parmi lesdits capteurs agencés sur ladite partie distale tandis que la partie distale de l'outil de traitement et l'échantillon de tissu sont tous les deux alignés simultanément avec ledit capteur, et tout en maintenant le support allongé en place pendant le traitement de tissu, et d'un échantillon de tissu situé au-delà de ladite extrémité distale du support allongé.

2. Dispositif selon la revendication 1, dans lequel le réseau de capteurs de caractérisation de tissu comprend en outre des capteurs agencés dans une relation espacée le long d'une région circonférentielle du support allongé à l'intérieur d'au moins une partie de ladite partie proximale.

3. Dispositif selon l'une quelconque des revendications précédentes, comprenant une partie poignée apte à être reliée à ou solidaire de l'extrémité proximale du support.

4. Dispositif selon la revendication 3, dans lequel la partie poignée est configurée pour être engagée avec une partie poignée d'un outil de traitement prédéterminé lorsque l'outil de traitement est introduit dans la partie creuse du support.

5. Dispositif selon l'une quelconque des revendications précédentes, comprenant un mécanisme de mouvement situé à l'extrémité proximale du support, ledit mécanisme de mouvement étant configuré et actionnable pour réaliser un déplacement relatif entre le support et un outil de traitement porté par celui-ci.

6. Dispositif selon la revendication 5, dans lequel le mécanisme de mouvement est situé à l'intérieur d'une partie poignée qui est apte à être reliée à ou solidaire de l'extrémité proximale du support.

7. Dispositif selon la revendication 5 ou 6, dans lequel le mécanisme de mouvement est **caractérisé par** au moins un de ce qui suit :
(i) le mécanisme de mouvement comprend un ensemble d'alignement pour définir une position de référence pour le réseau de capteurs de caractérisation de tissu par rapport à un certain plan de référence défini par le dispositif, permettant ainsi de surveiller le repositionnement des capteurs de caractérisation de tissu causé par un mouvement du support par rapport au plan de référence ;
(ii) le mécanisme de mouvement comprend un système de verrouillage configuré pour maintenir ledit réseau de capteurs dans des positions fixes et verrouillées ;
(iii) le mécanisme de mouvement comprend un coulisseau configuré pour fournir ledit déplacement relatif permettant le déplacement sélectif d'un outil de traitement, porté par le support, entre des positions non-fonctionnelle et fonctionnelle ;
(iv) le mécanisme de mouvement est configuré pour transformer un mouvement de rotation en le déplacement relatif linéaire entre le support et un outil de traitement porté par celui-ci ;
(v) le mécanisme de mouvement comprend une configuration à pignon et crémaillère.

8. Dispositif selon la revendication 7, dans lequel le mécanisme de mouvement comprend l'ensemble d'alignement, ledit ensemble d'alignement comprenant au moins un de ce qui suit : (a) une unité de détection configurée et actionnable pour identifier une position du réseau de capteurs par rapport audit plan de référence et pour générer des données indicatives de celle-ci ; (b) une monture en forme de L qui, par son premier bras d'une longueur donnée, est reliée de manière mobile à la partie poignée et, par son autre bras, est reliée au support, le plan de référence étant défini par un bord distal de la partie poignée, permettant ainsi de surveiller le repositionnement des capteurs de caractérisation de tissu causé par un mouvement de la monture par rapport au plan de référence.

9. Dispositif selon l'une quelconque des revendications précédentes, configuré sous la forme d'un ensemble à deux parties comprenant un premier ensemble et un second ensemble, les premier et second ensembles pouvant être fixés l'un à l'autre de manière amovible, l'ensemble à deux parties ayant l'une des configurations suivantes : (i) ledit premier ensemble porte la sonde de caractérisation de tissu et le second ensemble est configuré pour porter un outil de traitement ; (ii) l'un ou l'autre desdits premier et second ensembles est configuré pour porter la sonde de caractérisation de tissu et un outil de traitement.

10. Dispositif selon l'une quelconque des revendications précédentes, comprenant une unité de commande configurée pour recevoir et analyser les signaux de caractérisation de tissu provenant des capteurs et utiliser des données indicatives de l'emplacement de chacun des capteurs respectifs pour déterminer un emplacement d'un échantillon de tissu anormal, permettant ainsi un traitement conséquent de l'échantillon de tissu anormal par un outil de traitement porté par le support.

11. Dispositif selon la revendication 10, dans lequel l'unité de commande a au moins une des configurations suivantes : comprend une interface utilisateur graphique configurée pour présenter des informations relatives aux signaux reçus à partir de tous les capteurs, fournissant ainsi à un opérateur des informations concernant le type de tissu aux emplacements des capteurs, et facilitant l'analyse d'un emplacement et de l'étendue du tissu à traiter ; et est configurée pour recevoir, à partir d'une ou plusieurs modalités d'imagerie, des données d'imagerie de la position de sonde de caractérisation de tissu par rapport à un tissu mesuré et fusionner les données d'imagerie aux données mesurées, pour ainsi identifier des emplacements des capteurs sur une image fusionnée.

12. Dispositif selon la revendication 11, dans lequel l'unité de commande comprend ladite interface utilisateur graphique, l'interface utilisateur graphique étant configurée pour présenter un réseau d'emplacements de mesure correspondant au réseau de capteurs disposés le long au moins dudit axe qui est un axe de mesure, et présenter un réseau correspondant de données mesurées en association avec le réseau d'emplacements de mesure, ledit réseau de données mesurées indiquant les signaux reçus respectivement à partir du réseau de capteurs, fournissant ainsi à un opérateur des informations concernant un profil de tissu mesuré le long d'une masse de tissu alignée avec le réseau de capteurs.

13. Dispositif selon la revendication 12, dans lequel ladite interface utilisateur graphique est configurée pour afficher ledit réseau d'emplacements de mesure sous la forme du réseau de données mesurées chacune représentée par l'un de ce qui suit : (1) une valeur respective correspondant à une propriété de tissu détectée par le capteur respectif, un réseau des valeurs définissant le profil de la propriété de tissu mesurée à l'intérieur d'une région alignée avec le réseau de capteurs ; (2) une figure respective ayant une géométrie ou taille correspondant à une propriété de tissu détectée par le capteur respectif, un réseau des figures définissant le profil de la propriété de tissu mesurée à l'intérieur d'une région alignée avec le réseau de capteurs ; (3) une valeur et une figure respectives, une géométrie ou taille de la figure et la valeur correspondant à une propriété de tissu détectée par le capteur respectif, un réseau des figures et valeurs définissant le profil de la propriété de tissu mesurée à l'intérieur d'une région alignée avec le réseau de capteurs.

14. Dispositif selon la revendication 13, dans lequel ladite interface utilisateur graphique a au moins une des configurations suivantes : est configurée pour afficher lesdites figures sous la forme de figures colorées, de telle sorte que les figures correspondant aux mêmes données mesurées ont la même couleur et des figures correspondant à des données mesurées différentes ont des couleurs différentes ; est configurée pour afficher des données indiquant une valeur de seuil pour la propriété de tissu mesurée ; et est configurée pour afficher ledit réseau d'emplacements de mesure sous la forme du réseau de données mesurées chacune représentée par une région colorée, de telle sorte que les régions correspondant aux mêmes données mesurées ont la même couleur et des régions correspondant à des données mesurées différentes ont des couleurs différentes.
